# EUROPEAN PATENT APPLICATION

(11) **EP 4 516 788 A1**
(43) Date of publication of application: **05.03.2025**
(21) Application number: 23795535.6
(22) Date of filing: 27.04.2023
(51) Int. Cl.: C07D 403/04, A61P 19/06, A61K 31/415, A61K 31/416, A61K 31/4192

(54) **COMPOUND CAPABLE OF BEING USED FOR GOUT**

(30) Priority: 27.04.2022 CN 202210458821
(71) Applicant: Atom Therapeutics Co., Ltd, Hangzhou, Zhejiang 310018 (CN)
(72) Inventor: SHI, Dongfang, Suzhou, Jiangsu 215123 (CN); FU, Zhangjin, Suzhou, Jiangsu 215123 (CN); YANG, Yan, Suzhou, Jiangsu 215123 (CN); LI, Haiming, Suzhou, Jiangsu 215123 (CN); YUAN, Chengyi, Suzhou, Jiangsu 215123 (CN)
(74) Representative: Cabinet Beau de Loménie
(86) International application number: PCT/CN2023/091109
(87) International publication number: WO 2023/208103

(57) **Abstract**

The present invention discloses a class of compounds for gout, which is a compound as represented by general formula (I) or a pharmaceutically acceptable salt thereof, can reduce the serum uric acid level in a rat model of hyperuricemia, and has potential application value in aspects such as an anti-gout medicament and an anti-hyperuricemia medicament.

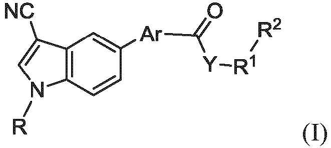

## Description

### TECHNICAL FIELD

The present invention belongs to the field of medicinal chemistry, and specifically relates to the compounds which can be used for gout.

### BACKGROUND

Gout is a metabolic disease caused by increased uric acid production and/or decreased uric acid excretion in the body, which leads to an increased serum uric acid (sUA) level, forming monosodium urate crystals that are deposited in joints, surrounding tissues and kidneys, thereby causing autoinflammatory reactions and changes in joint morphology. Gout is the most prevalent inflammatory arthropathy in adult men, with an increasing prevalence in developed countries, and has become a serious metabolic disease that threatens human health.

There are currently more than 55 million gout patients worldwide, and the number of patients with hyperuricemia is far more than 200 million. Over the past two decades, with the improvement of people's living standards, changes in dietary structure, excessive intake of high-purine foods, and a large increase in the number of patients with hyperuricemia and gout, the gout market has shown a trend of substantial growth. Data shows that the global gout drug market was 15.65 billion in 2018 and is expected to reach 36.15 billion in 2025. Gout is often associated with a variety of diseases such as hypertension, obesity, cardiovascular disease, diabetes, and chronic kidney diseases, and these comorbidities complicate gout treatment and increase the risk of premature death (Khanna D, Fitzgerald JD, Khanna PP, et al., American College of Rheumatology Guidelines for Management of Gout. Part 1: Systematic Nonpharmacologic and Pharmacologic Therapeutic Approaches to Hyperuricemia[J]. Arthritis Care & Research, 2012, 64(10): 1432-1446). Patients with severe gout may suffer from joint disability and renal insufficiency, which seriously affects their quality of life and health.

Xanthine oxidase (XO) is an important target for drug treatment of hyperuricemia and gout, and catalyzes the oxidation of hypoxanthine to xanthine and the oxidation of xanthine to uric acid, respectively. Inhibiting XO can reduce the synthesis of uric acid, thereby reducing the concentration of uric acid in serum. With the analysis of the crystal structure of xanthine oxidase, the application of computer-aided drug design and high-throughput screening, many XO inhibitors have emerged in recent years. Patent CN102574839A discloses a novel compound as an inhibitor of xanthine oxidase, wherein a candidate compound LC350189 is being developed and is in a phase 2 clinical stage, and the phase 2 clinical study also proves that it reduces the full therapeutic effect of sUA. However, currently only three drugs (allopurinol, Febuxostat andTopiroxostat) have been approved for marketing, and the drugs under development in clinical trials still have problems with efficacy or safety.

The prodrug strategy is an effective drug design method that modifies drug molecules that are known to have biological activity but have certain disadvantages, adds precursor groups, and forms chemical substances that can be activated by enzymes or chemical reactions in vivo. Prodrugs generally have no physiological activity, or very low activity, but may be enzymatically or non-enzymatically converted to physiologically active drugs. Compared with original drugs, prodrugs not only maintain or enhance the efficacy of the original drugs, but also overcome the defect of the original drugs, and improve the clinical efficacy thereof. Prodrugs can be used to increase the solubility of drugs, improve the bioavailability, improve the transfer properties and the pharmacokinetic properties, etc. Currently, nearly 10% of drugs on the global market are prodrugs, and in 2008 alone, about 30% of small molecule drugs were prodrugs. For example, angiotensin-converting enzyme inhibitors such as Enalapril, Benazepril, and Ramipril are hydrolyzed in the body to produce corresponding dicarboxylic acid metabolites, thereby exerting a hypotensive effect; some statins such as Lovastatin and Simvastatin are prodrugs having a cyclic structure, and have hydroxymethylglutaryl-CoA reductase inhibitory activity only after the ring-opening reaction; proton pump inhibitors, such as Omeprazole, Lansoprazole and Imeprazole, all require to be activated in the acidic environment of the stomach; and the antihistamine loratadine removes the ethyl formate group in vivo to generate the active metabolite desloratadine. In addition, many novel prodrugs are being developed. However, in actual research and development, the prodrug compounds still have many problems such as loss of activity or poor activity, and the designed prodrug compounds not corresponding to the designed target effect.

### SUMMARY

An object of the present invention is a compound having xanthine oxidase inhibitory activity.

Another object of the present invention is to provide use of the compound above in the medical field.

The object of the present invention can be achieved by the following measures.

A compound as represented by general formula (I) or a pharmaceutically acceptable salt thereof, 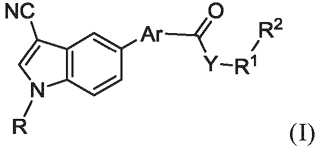 wherein,
R is C₁₋₆ alkyl, substituted C₁₋₆ alkyl, C₃₋₆ cycloalkyl, substituted C₃₋₆ cycloalkyl, C₃₋₆ heterocycloalkyl or substituted C₃₋₆ heterocycloalkyl; wherein the substituent in each group involved in R is one or more selected from deuterium, cyano, nitro, halo, C₁₋₆ alkyl, C₁₋₆ alkoxy, C₃₋₆ cycloalkyl or C₃₋₆ heterocycloalkyl;
Ar is a substituted or unsubstituted group of: 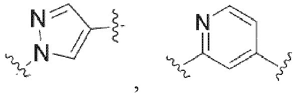 or 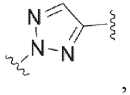 and the substituent in the Ar group is one or more selected from deuterium, hydroxyl, halo, C₁₋₄alkyl or C₁₋₄alkoxy;
Y is O or NR³,
R¹ is a linking bond or substituted or unsubstituted C₁₋₆ alkylene or substituted or unsubstituted C₂₋₁₂ alkenylene, and the substituent in the R¹ group is one or more selected from deuterium, hydroxyl, amino, cyano, halo, C₁₋₄ alkyl or C₁₋₄ alkoxy;
R² is one or more of hydrogen, nitrooxy, carboxyl or the following substituted or unsubstituted groups: dioxol-2-one, C₄₋₁₂fused heteroarylcyclyl, C₄₋₁₆ fused heteroarylcyclylpyrazolylcarbonyloxy, C₄₋₁₆ fused heteroarylcyclylpyridylcarbonyloxy, C₄₋₁₆ fused heteroarylcyclyltriazolylcarbonyloxy, C₂₋₆ ester, pyridyl, phenyl, C₁₋₆ alkoxy, C₂₋₂₀ alkenyl, C₂₋₂₀ alkynyl, C₂₋₈ alkylcarbonyloxy or C₂₋₈ alkoxycarbonyloxy, and the substituent in the R² group is one or more selected from deuterium, hydroxyl, amino, cyano, halo, C₁₋₄ alkyl, halo C₁₋₄ alkyl, nitrooxy-substituted C₁₋₄ alkyl or C₁₋₄ alkoxy; and the C₄₋₁₆ fused heteroarylcyclyl in the R² group does not contain indazolyl; and
R³ is hydrogen or C₁₋₆ alkyl.

In a preferred embodiment, Ar is a substituted or unsubstituted group of: 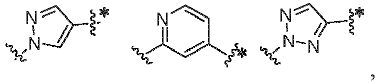 wherein "*" is the attachment site to C=O.

In a preferred embodiment, the compound is selected from compounds of formula (II), (III) or (IV),

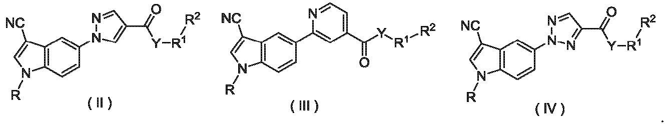

In a preferred embodiment, Y is O or NH.

In a preferred embodiment, R is C₃₋₆ alkyl, substituted C₁₋₆ alkyl, C₃₋₆ cycloalkyl, substituted C₃₋₆ cycloalkyl, C₃₋₆ heterocycloalkyl or substituted C₃₋₆ heterocycloalkyl; wherein the substituent in each group involved in R is one or more selected fromdeuterium, cyano, nitro, halo, C₁₋₅ alkyl, C₁₋₅ alkoxy or C₃₋₆ cycloalkyl.

In a preferred embodiment, R is C₃₋₆ alkyl, substituted C₁₋₆ alkyl, C₃₋₆ cycloalkyl, substituted C₃₋₆ cycloalkyl, tetrahydrofuran, substituted tetrahydrofuran, tetrahydrothiophene, substituted tetrahydrothiophene, tetrahydropyrrole or substituted tetrahydropyrrole; wherein the substituent in each group involved in R is one or more selected fromdeuterium, cyano, nitro, halo, C₁₋₅ alkyl, C₁₋₅ alkoxy or C₃₋₆ cycloalkyl.

In a preferred embodiment, R is C₃₋₆ alkyl, substituted C₁₋₃ alkyl, C₃₋₆ cycloalkyl or substituted C₃₋₆ cycloalkyl, and the substituent in R group is selected from deuterium, halogen or C₃₋₆ cycloalkyl.

In a preferred embodiment, R is C₃₋₆ alkyl or C₃₋₆ cycloalkyl.

In a preferred embodiment, R is n-propyl, isopropyl, n-butyl, isobutyl, cyclopropyl, cyclobutyl or cyclopentyl.

In a preferred embodiment, R¹ is a linking bond or substituted or unsubstituted C₁₋₄ alkylene or substituted or unsubstituted C₂₋₁₂ alkenylene, and the substituent in the R¹ group is one or more selected fromdeuterium, amino, cyano, halogen or C₁₋₄ alkoxy.

In a preferred embodiment, R² is one or more of hydrogen, nitrooxy, carboxyl orthe following substituted or unsubstituted groups: dioxol-2-one, indazolyl, quinolinyl, isoquinolinyl, indolyl, benzofuranyl, purinyl, quinolinylpyrazolylcarbonyloxy, isoquinolinylpyrazolylcarbonyloxy, indolylpyrazolylcarbonyloxy, benzofuranylpyrazolylcarbonyloxy, purinylpyrazolylcarbonyloxy, quinolinylpyridylcarbonyloxy, isoquinolinylpyridylcarbonyloxy, indolylpyridylcarbonyloxy, benzofuranylpyridylcarbonyloxy, purinylpyridylcarbonyloxy, quinolyltriazolylcarbonyloxy, isoquinolyltriazolylcarbonyloxy, indolyltriazolylcarbonyloxy, benzofuranyltriazolylcarbonyloxy, purinyltriazolylcarbonyloxy, C₂₋₆ ester, pyridyl, phenyl, C₁₋₆ alkoxy, C₆₋₂₀ alkenyl, C₆₋₂₀ alkynyl, C₂₋₈ alkylcarbonyloxy or C₂₋₈ alkoxycarbonyloxy, and the substituent in the R² group is one or more selected fromdeuterium, hydroxyl, amino, cyano, halo, C₁₋₄ alkyl, halo C₁₋₄ alkyl, nitrooxy-substituted C₁₋₄ alkyl or C₁₋₄ alkoxy.

In a preferred embodiment, R² is one or more of hydrogen, nitrooxy, carboxyl or the following substituted or unsubstituted groups: dioxol-2-one, indolylpyrazolylcarbonyloxy, indolylpyridylcarbonyloxy, indolyltriazolylcarbonyloxy, C₂₋₆ester, pyridyl, phenyl, C₁₋₆alkoxy, C₆₋₂₀alkenyl, C₂₋₈alkylcarbonyloxy or C₂₋₈alkoxycarbonyloxy, and the substituent in the R² group is one or more selected from deuterium, hydroxyl, amino, cyano, halo, C₁₋₄ alkyl, halo C₁₋₄ alkyl, nitrooxy-substituted C₁₋₄ alkyl or C₁₋₄ alkoxy.

In a preferred embodiment, R²is one or more of hydrogen, nitrooxy, carboxyl or the following substituted or unsubstituted groups: dioxol-2-onyl, indolylpyrazolylcarbonyloxy, indolylpyridylcarbonyloxy, C₂₋₄ester, phenyl, C₁₋₄alkoxy, C₆₋₁₄alkenyl, C₂₋₈alkylcarbonyloxy or C₂₋₈alkoxycarbonyloxy, and the substituent in the R² group is one or more selected fromdeuterium, hydroxyl, amino, cyano, halo, C₁₋₄ alkyl, nitrooxy- substituted C₁₋₄ alkyl or C₁₋₄ alkoxy.

In a preferred embodiment, the compound of the invention is selected from:

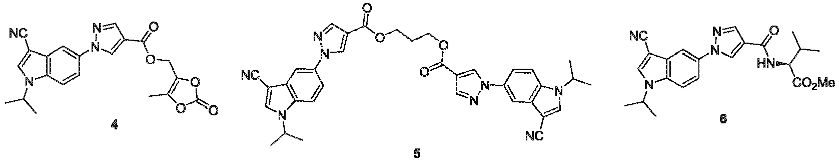

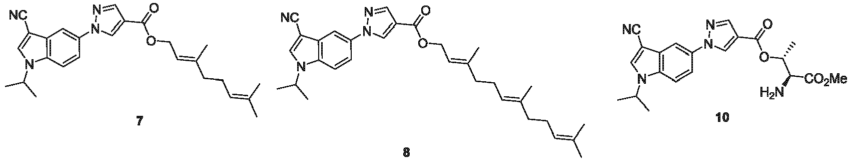

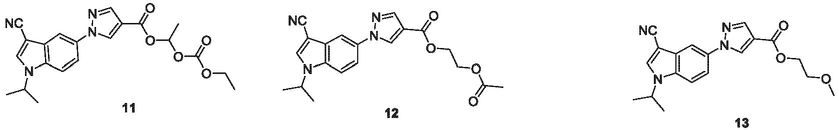

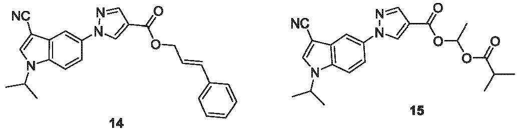

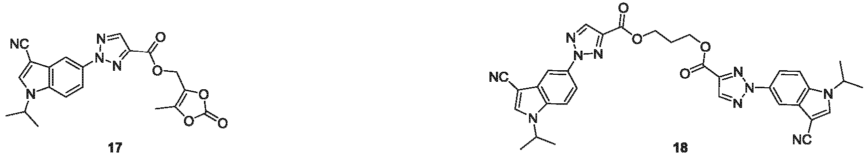

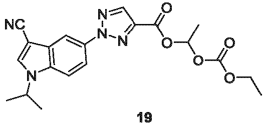

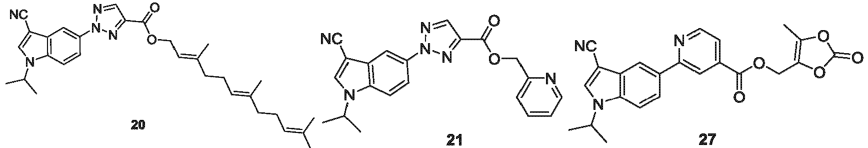

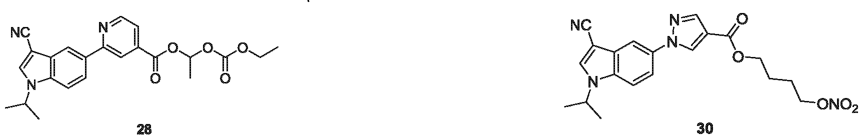

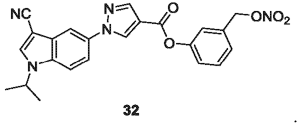

The invention also relates to a pharmaceutical composition, which uses the compound or the pharmaceutically acceptable salt thereof involved in the present application as an active substance, and uses a pharmaceutically acceptable excipient.

The compound or the pharmaceutically acceptable salt thereof of the invention can be used in the preparation of a xanthine oxidase inhibitor medicament, particularly in the preparation of an anti-gout medicament or an anti-hyperuricemia medicament.

The groups specified in the invention, if not explicitly defined otherwise, have the following meanings.

"H", i.e. hydrogen, refers to protium (1H), which is the main stable isotope of hydrogen element.

"D", or "deuterium", refers to a stable isotope of hydrogen, also known as heavy hydrogen, with the element symbol D.

"Halogen" refers to fluorine atom, chlorine atom, bromine atom or iodine atom.

"Hydroxy" refers to -OH group.

"Amino" refers to -NH₂ group.

"Alkyl" refers to saturated aliphatic hydrocarbon groups containing 1-10 carbon atoms, including linear and branched chain groups (the numerical range mentioned in the present application, such as "1-10", means that the group, in this case alkyl,may contain 1 carbon atom, 2 carbon atoms, 3 carbon atoms, and so on, up to10 carbon atoms). Alkyl containing 1-4 carbon atoms is referred to as lower alkyl. When the lower alkyl has no substituent, it is called unsubstituted lower alkyl. The alkyl may be selected from C₁₋₆ alkyl, C₁₋₃ alkyl, C₁₋₄ alkyl, C₁₋₃ alkyl, C₁₋₂ alkyl, C₂₋₃ alkyl, C₂₋₄ alkyl, etc. Specific alkyl includes, but is not limited to, methyl, ethyl, propyl, 2-propyl, n-butyl, isobutyl, t-butyl, etc. The alkylmay be substituted or unsubstituted.

"Alkenyl" refers to hydrocarbon groups having one or more "C=C" and containing 2-30 carbon atoms, including both linear and branched chain groups (the numerical range mentioned in the present application, such as "2-10", means that the group, in this case alkenyl, may contain 2 carbon atoms, 3 carbon atoms, and so on, up to10 carbon atoms). The alkenyl may be selected from C₂₋₂₀ alkenyl, C₂₋₁₈ alkenyl, C₂₋₁₆ alkenyl, C₂₋₁₄ alkenyl, C₂₋₁₂ alkenyl, C₄₋₁₄ alkenyl, C₄₋₁₂ alkenyl, etc. Specific alkenyl includes, but is not limited to, ethenyl, propenyl, allyl, butenyl, isobutenyl, t-butenyl, 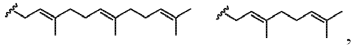 etc.

"Alkoxy" represents an-O-(unsubstituted alkyl) and-O-(unsubstituted cycloalkyl) group, and further represents-O-(unsubstituted alkyl). The alkyl therein may be selected from C₁₋₆ alkyl, C₁₋₅ alkyl, C₁₋₄ alkyl, C₁₋₃ alkyl, C₁₋₂ alkyl, C₂₋₃ alkyl, C₂₋₄ alkyl, etc. Representative examples include, but are not limited to, methoxy, ethoxy, propoxy, cyclopropoxy, etc.

"Dioxol-2-one" is a 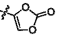 group.

"Pyrazolyl" refers to any one of

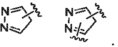

"Triazolyl" includes 1,2,3-triazolyl, wherein "1,2,3-triazolyl" is

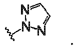

"Fused heteroarylcyclyl" refers to an aromatic group containing two or more fused rings and heteroatoms, including but not limited to indazolyl, quinolinyl, isoquinolinyl, indolyl, benzofuranyl, purinyl, acridinyl, etc.

"Carboxy" refers to -COOH group.

"Ester group" refers to a "-C(=O)-O-alkyl" group, wherein the alkyl may be selected from C₁₋₆ alkyl, C₁₋₅ alkyl, C₁₋₄ alkyl, C₁₋₃ alkyl, C₁₋₂ alkyl, C₂₋₃ alkyl, C₂₋₄ alkyl, etc. Representative examples include, but are not limited to, methyl formate group, ethyl formate group, n-propyl formate group, isopropyl formate group,etc. The substituted ester group means that hydrogen in the ester group is substituted with one substituent, or a plurality of hydrogen in the ester group are respectively substituted with same or different substituents.

"Heterocycloalkyl" refers to a saturated cyclic group containing 3-10 ring atoms, with one or more are heteroatoms selected from N, O andS. The numerical range mentioned in the present application, such as "3-6", means that the group, in this case heterocycloalkyl, may contain 3 carbon atoms, 4 carbon atoms, 5 carbon atoms, and so on, up to6 carbon atoms as ring atoms. The heterocycloalkyl can be selected from C₃₋₈ heterocycloalkyl, C₃₋₆ heterocycloalkyl, C₃₋₅ heterocycloalkyl, C₃₋₄ heterocycloalkyl, C₃₋₉ heterocycloalkyl, C₄₋₆ heterocycloalkyl, etc. Specific alkyl includes, but is not limited to, tetrahydrofuran, tetrahydropyrrole, tetrahydrothiophene, 1,4-dioxane, oxospiro[3,3]heptanyl, oxospiro[4,4]nonanyl, oxospiro[5,5]undecanyl, oxospiro[6,6]tridecyl, oxobicyclo[1,1,1]pentanyl, oxobicyclo[2,2,2]octanyl, oxobicyclo[3,2,1]octanyl, azaspiro[3,3]heptanyl, azaspiro[4,4]nonanyl, azaspiro[5,5]undecanyl, azaspiro[6,6]tridecyl, azabicyclo[1,1,1]pentanyl, azabicyclo[2,2,2]octanyl, azabicyclo[3,2,1]octanyl, etc. The heterocycloalkyl may be substituted or unsubstituted.

"C₄₋₁₆ fused heteroarylpyrazolylcarbonyloxy" refers to an -O-C(=O)-pyrazolyl-fused heteroarylcyclyl group containing 4-16 carbon atoms. A specific exampleincludes indolylpyrazolylcarbonyloxy

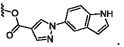

"C₂₋₈ alkoxycarbonyloxy" refers to an -O-C(=O)-O-alkyl group containing 2-8 carbon atoms.

"C₄₋₁₆ fused heteroarylcyclylpyridylcarbonyloxy" refers to an -O-C(=O)-pyridyl-fused heteroarylcyclyl group containing 4-16 carbon atoms. A specific exampleincludes indolylpyridylcarbonyloxy

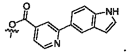

"C₄₋₁₆fused heteroaryltriazolylcarbonyloxy" refers to an -O-C(=O)-triazolyl-fused heteroarylcyclyl group containing 4-16 carbon atoms. A specific exampleincludes indolyltriazolylcarbonyloxy

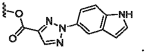

"Linking bond" means that groups on two ends are directly linked by a covalent bond. Taking the group fragment Y-R¹-R² as an example, when R¹ is a linking bond, the group fragment is Y-R².

"Nitrooxy" refers to-ONO₂ group.

"Pharmaceutically acceptable salt" is a salt formed by the compound of general formula (I)with an organic or inorganic acid, and represents those salts that retain the biological effectiveness and properties of the parent compound. Such salts include, but are not limited to:
(1) salts formed with an acid, obtained by reaction of a free base of the parent compound with an inorganic acid or an organic acid, wherein the inorganic acid is, for example, but not limited to, hydrochloric acid, hydrobromic acid, nitric acid, phosphoric acid, metaphosphoric acid, sulfuric acid, sulfurous acid, perchloric acid or the like; and the organic acid is, for example, but not limited to, acetic acid, propionic acid, acrylic acid, oxalic acid, (D) or (L) malic acid, fumaric acid, maleic acid, hydroxybenzoic acid, γ-hydroxybutyric acid, methoxybenzoic acid, phthalic acid, methanesulfonic acid, ethanesulfonic acid, naphthalene-1-sulfonic acid, naphthalene-2-sulfonic acid, p-toluenesulfonic acid, salicylic acid, tartaric acid, citric acid, lactic acid, mandelic acid, succinic acid or malonic acid or the like; and
(2) salts formed when an acidic proton in the parent compound is replaced by a metal ion or the parent compound is coordinated with an organic base, wherein the metal ion, is, for example, an alkali metal ion, an alkaline earth metal ion or an aluminum ion, and the organic base is, for example, ethanolamine, diethanolamine, triethanolamine, tromethamine, N-methylglucamine or the like.

"Pharmaceutical composition" refers to a mixture of one or more compounds described herein, or a pharmaceutically acceptable salt and a prodrug thereof, with other chemical ingredients, such as a pharmaceutically acceptable carrier and an excipient. The purpose of the pharmaceutical composition is to facilitate administration of the compound to an organism.

The invention further sets forth a pharmaceutical composition comprising any one of the compounds described above, a pharmaceutically acceptable salt thereof or a hydrolyzable prodrug thereof, together with other pharmaceutically active ingredients.

In the invention, any of the described compounds and pharmaceutically acceptable salts thereof can be formulated into any dosage form which is clinically or pharmaceutically acceptable in a manner known in the art. For oral administration, conventional solid formulations such as tablets, capsules, pills, granules, etc. can be prepared; oral liquid formulations such as oral solutions, oral suspensions, syrups, etc. can also be prepared. When preparing oral formulations, suitable fillers, binders, disintegrating agents, lubricants, etc. can be added. For parenteral administration, injections can be prepared, including injection solutions, sterile powders for injection, and concentrated solutions for injection. When preparing injections, conventional methods in the existing pharmaceutical field can be used, and when preparing the injections, additives may not be added, or appropriate additives may be added according to the properties of the drug.

The compound provided in the invention can significantly reduce the serum uric acid level in a rat model of hyperuricemia, and has potential application value in aspects such as anti-gout medicaments and anti-hyperuricemia medicaments. Because Febuxostat can cause severe sudden cardiac death, severe renal toxicity and liver toxicity, the compound provided by the present invention may have certain advantages in reducing drug toxicity and have good prospects for drug development.

### DETAILED DESCRIPTION

The present invention is further described below with reference to embodiments, but the scope of protection of the present invention is not limited to the following embodiments.

### Example 1: Synthesis of (5-methyl-2-oxo-1,3-dioxol-4-yl)methyl 1-(3-cyano-1-isopropyl-1H-indol-5-yl)-1H-pyrazole-4-carboxylate (4)

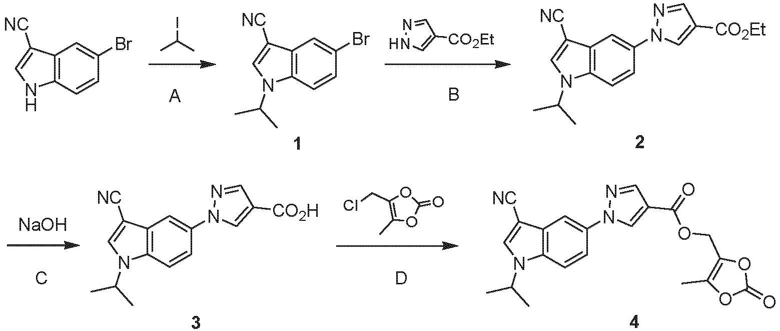

Step A: A mixture comprising 5-bromo-1H-indole-3-carbonitrile (10.0 g, 45.2 mmol), iodoisopropane (30.8 g, 181 mmol), cesium carbonate (29.5 g, 90.5 mmol) and acetonitrile (100 mL) was stirred at 80°C for 3 hours, cooled to room temperature, and filtered to remove insolubles. The filter cake was rinsed with ethyl acetate (200 mL). The solvent was evaporated under reduced pressure. The product was purified by column chromatography (200-300 mesh silica gel, ethyl acetate: petroleum ether = 1:15-1:3 elution) to give 5-bromo-1-isopropyl-1H-indole-3-carbonitrile **(1)** (11.6 g). The yield was 97.5%.

Step B: A mixture comprising ethyl 1H-pyrazole-4-carboxylate (3.20 g, 22.8 mmol), compound **1** (3.03 g, 11.5 mmol), potassium carbonate (3.15 g, 22.8 mmol), copper iodide (2.17 g, 11.4 mmol), (1S,2S)-1,2-diaminocyclohexane (1.01 g, 11.4 mmol) and DMF (50 mL) was stirred under nitrogen at 110°C overnight. After cooling to room temperature, ethyl acetate (100 mL) was added. The mixture was washed with saturated brine (50 mL x 3), and dried over anhydrous sodium sulfate. The solvent was evaporated under reduced pressure. The product was purified by column chromatography (200-300 mesh silica gel, ethyl acetate: petroleum ether = 1:30-1:4 elution) to give ethyl 1-(3-cyano-1-isopropyl-1H-indol-5-yl)-1H-pyrazole-4-carboxylate **(2)** (3.10 g). The yield was 83.6%.

Step C: A mixture comprising compound **2** (600 mg, 1.86 mmol), a 2M sodium hydroxide solution (12 mL), methanol (4 mL) and THF (4 mL) was stirred at 30°C for 1 hour. A portion of the solvent was evaporated under reduced pressure. Water (10 mL) was added. Ethyl acetate (10 mL) was used for extraction, and the product was in the aqueous phase. The pH of the aqueous phase was adjusted to4-5 bya 2M citric acid solution. The filter cake was recrystallized in methanol to give 1-(3-cyano-1-isopropyl-1H-indol-5-yl)-1H-pyrazole-4-carboxylic acid **(3)** (400 mg). The yield was 73.1%.

Step D: A mixture comprising compound **3** (150 mg, 0.510 mmol), 4-chloromethyl-5-methyl-1,3-dioxol-2-one (91 mg, 0.613 mmol), potassium carbonate (140 mg, 1.01 mmol), potassium iodide (110 mg, 0.663 mmol) and DMF (5 mL) was stirred overnight at room temperature. Water (20 mL) was added for filtration. The filter cake was purified by column chromatography (200-300 mesh silica gel, dichloromethane elution) to give (5-methyl-2-oxo-1,3-dioxol-4-yl)methyl 1-(3-cyano-1-isopropyl-1H-indol-5-yl)-1H-pyrazole-4-carboxylate **(4).** ¹H NMR (DMSO-d₆,400 MHz): δ 9.30 (s,1H),8.58 (s,1H),8.23 (s,1H),8.21 (s,1H),7.96-7.90 (m,2H),5.19 (s,2H),4.96-4.89 (m,1H),2.23 (s,3H),1.51 (d, J = 6.4 Hz,6H). MS (ESI, m/z):407.1 [M+H]⁺.

### Example 2: Synthesis of bis[1-(3-cyano-1-isopropyl-1H-indol-5-yl)-1H-pyrazole-4-carboxylic acid]propan-1,3-diester (5)

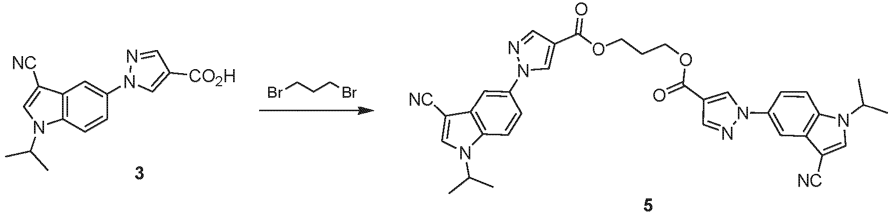

A mixture comprising compound **3** (150 mg, 0.510 mmol), 1,3-dibromopropane (51 mg, 0.253 mmol), potassium carbonate (141 mg, 1.02 mmol), potassium iodide (110 mg, 0.663 mmol) and DMF (5 mL) was stirred at 30°C for 48 hours. Water (20 mL) was added for filtration. The filter cake was purified by column chromatography (200-300 mesh silica gel, dichloromethane: petroleum ether = 1:1 elution) to give bis[1-(3-cyano-1-isopropyl-1H-indol-5-yl)-1H-pyrazole-4-carboxylic acid]propan-1,3-diester (5) (59.4 mg). The yield was 37.1%. ¹H NMR (DMSO-d₆,400 MHz): δ 9.19 (s,2H),8.55 (s,2H),8.14-8.12 (m,4H),7.86-7.84 (m,4H),4.93-4.86 (m,2H),4.43 (t, J = 6.0 Hz,4H),2.16 (t, J = 6.0 Hz,2H),1.49 (d, J = 6.4 Hz,12H). MS (ESI, m/z):629.2 [M+H]⁺).

### Example 3: Synthesis of [1-(3-cyano-1-isopropyl-1H-indol-5-yl)-1H-pyrazole-4-carbonyl]-L-valine methyl ester (6)

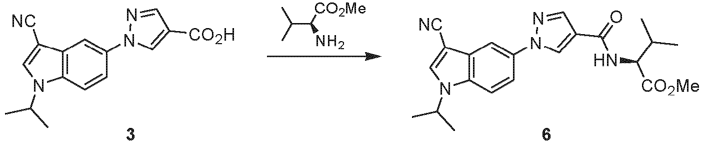

A mixture comprising compound 3 (70 mg, 0.238 mmol), L-valine methyl ester hydrochloride (47.8 mg, 0.285 mmol), diisopropylethylamine (77 mg, 0.596 mmol), HBTU (135 mg, 0.356 mmol) and DMF (5 mL) was stirred overnight at room temperature. Water (20 mL) was added. Ethyl acetate (30 mL x 2) was used for extraction. The combined organic phase was washed with saturated brine (15 mL x 3) and dried over anhydrous sodium sulfate. The solvent was evaporated under reduced pressure. The product was purified by column chromatography (200-300 mesh silica gel, petroleum ether: dichloromethane: triethylamine = 200: 100: 1 elution) to give [1-(3-cyano-1-isopropyl-1H-indol-5-yl)-1H-pyrazole-4-carbonyl]-L-valine methyl ester **(6)** (78.5 mg). The yield was 80.9%. ¹H NMR (DMSO-d₆,400 MHz): δ 9.16 (s,1H),8.57 (s,1H),8.29 (d, J = 8.0 Hz,1H),8.22 (s,1H),8.06 (s,1H),7.94-7.86 (m,2H),4.95-4.89 (m,1H),4.38-4.34 (m,1H),3.67 (s,3H),2.18-2.12 (m,1H),1.51 (d, J = 6.4 Hz,6H),0.99 (d, J = 6.8 Hz,3H),0.95 (d, J = 6.8 Hz,3H). MS (ESI, m/z):408.2 [M+H]⁺.

### Example 4: Synthesis of (3,7-dimethyloctan-2,6-dien-1-yl) 1-(3-cyano-1-isopropyl-1H-indol-5-yl)-1H-pyrazole-4-carboxylate (7)

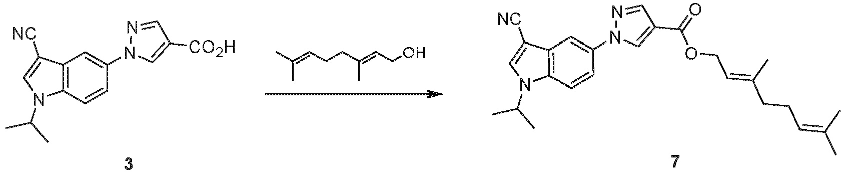

A mixture comprising compound **3** (70 mg, 0.238 mmol), 3,7-dimethyloctan-2,6-dien-1-ol (44 mg, 0.285 mmol), DCC (74 mg, 0.359 mmol), DMAP (3 mg, 0.0246 mmol) and dichloromethane (5 mL) was stirred overnight at room temperature. Insolubles were removed by filtration, and the filter cake was rinsed with dichloromethane (5 mL). The solvent was evaporated under reduced pressure. The product was purified by column chromatography (200-300 mesh silica gel, petroleum ether: dichloromethane: triethylamine = 300:100:1 elution) to give (3,7-dimethyloctan-2,6-dien-1-yl) 1-(3-cyano-1-isopropyl-1H-indol-5-yl)-1H-pyrazole-4-carboxylate (7) (53 mg). The yield was 51.7%. ¹H NMR (DMSO-d₆,400 MHz): δ 9.22 (s,1H),8.57 (s,1H),8.22 (d, J = 1.6 Hz,1H),8.14 (s,1H),7.95-7.89 (m,2H),5.44-5.41 (m,1H),5.08-5.06 (m,1H),4.95-4.89 (m,1H),4.77 (d, J = 7.2 Hz,2H),2.10-2.05 (m,4H),1.74 (s,3H),1.63 (s,3H),1.57 (s,3H),1.51 (s,3H),1.50 (s,3H). MS (ESI, m/z):431.2 [M+H]⁺.

### Example 5: Synthesis of (3,7,11-trimethyldodecyl-2,6,10-trien-1-yl)1-(3-cyano-1-isopropyl-1H-indol-5-yl)-1H-pyrazole-4-carboxylate (8)

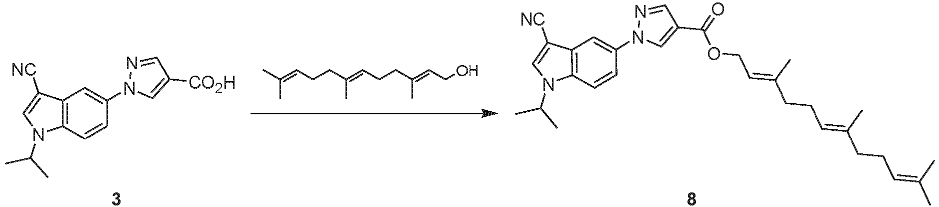

Compound 3 and 3,7,11-trimethyldodecyl-2,6,10-trien-1-ol were used as raw materials, and reference can be made to Example 4 for the experimental procedure for synthesizing compound **8.** ¹H NMR (DMSO-d₆,400 MHz): δ 9.20 (s,1H),8.57 (s,1H),8.21 (s,1H),8.13 (d, J = 2.4 Hz,1H),7.95-7.89 (m,2H),5.43-5.40 (m,1H),5.10-5.02 (m,2H),4.95-4.89 (m,1H),4.78-4.73 (m,2H),2.11-1.91 (m,8H),1.74 (s,3H),1.64-1.50 (m,15H). MS (ESI, m/z):499.2 [M+H]⁺.

### Example 6: Synthesis of [(2R,3S)-3-amino-4-methoxy-4-oxobutan-2-yl] 1-(3-cyano-1-isopropyl-1H-indol-5-yl)-1H-pyrazole-4-carboxylate (10)

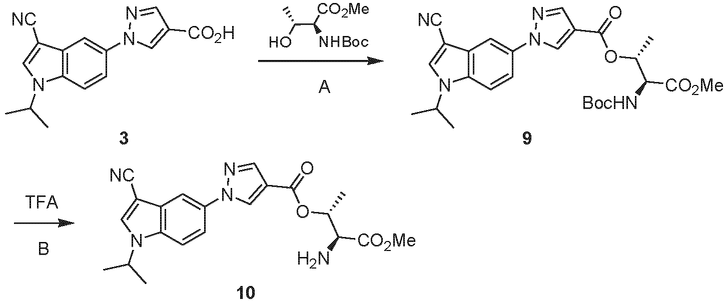

Step A: A mixture comprising compound **3** (120 mg, 0.408 mmol), Boc-L-threonine methyl ester (114 mg, 0.489 mmol), DCC (168 mg, 0.814 mmol) and dichloromethane (4 mL) was stirred overnight at room temperature. Insolubles were removed by filtration, and the filter cake was rinsed with dichloromethane (5 mL). The solvent was evaporated under reduced pressure. The product was purified by column chromatography (200-300 mesh silica gel, petroleum ether: dichloromethane: triethylamine = 300: 100: 1 elution) to give [(2R,3S)-3-(Boc-amino)-4-methoxy-4-oxobutan-2-yl] 1-(3-cyano-1-isopropyl-1H-indol-5-yl)-1H-pyrazole-4-carboxylate **(9)** (218 mg). The yield was 100%.

Step B: A solution of compound **9** (218 mg, 0.427 mmol) and trifluoroacetic acid (0.4 mL) in dichloromethane (4 mL) was stirred at room temperature overnight. Water (20 mL) was added. The pH was adjusted to 7-8 by a saturated sodium bicarbonate solution. Dichloromethane (20 mL x 2) was used for extraction. The combined organic phase was washed with saturated brine (10 mL x 2), and dried over anhydrous sodium sulfate. The solvent was evaporated under reduced pressure, and the resulting product was recrystallized in ethyl acetate/petroleum ether to give [(2R,3S)-3-amino-4-methoxy-4-oxobutan-2-yl] 1-(3-cyano-1-isopropyl-1H-indol-5-yl)-1H-pyrazole-4-carboxylate **(10).** ¹H NMR (DMSO-d₆,400 MHz): δ 9.23 (s,1H),8.58 (s,1H),8.18-8.16 (m,2H),7.93-7.92 (m,2H),5.32-5.30 (m,1H),4.94-4.91 (m,1H),3.61-3.58 (m,4H),1.51 (d, J = 6.4 Hz,6H),1.35 (d, J = 6.4 Hz,3H). MS (ESI, m/z):410.1 [M+H]⁺.

### Example 7: Synthesis of {1-[(ethoxycarbonyl) oxy]}ethyl 1-(3-cyano-1-isopropyl-1H-indol-5-yl)-1H-pyrazole-4-carboxylate (11)

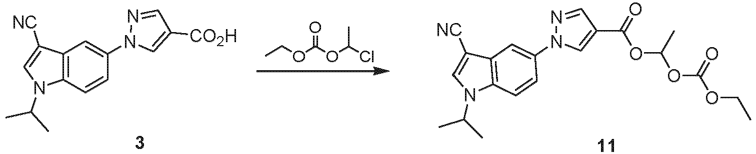

A mixture comprising compound **3** (100 mg, 0.340 mmol), potassium carbonate (93 mg, 0.673 mmol), 1-chloroethyl ethyl carbonate (78 mg, 0.511 mmol), potassium iodide (73 mg, 0.440 mmol) and DMF (2 mL) was stirred at 40°C for 48 hours. Water (20 mL) was added. Ethyl acetate (20 mL x 2) was used for extraction. The combined organic phase was successively washed with water (10 mL x 2) and saturated brine (10 mL), and dried over anhydrous sodium sulfate. The solvent was evaporated under reduced pressure. The product was purified by column chromatography (200-300 mesh silica gel, ethyl acetate: petroleum ether=1:10 elution) to give {1-[(ethoxycarbonyl)oxy]} ethyl 1-(3-cyano-1-isopropyl-1H-indol-5-yl)-1H-pyrazole-4-carboxylate **(11)**(51 mg). The yield was 36.5%. ¹H NMR (DMSO-d₆,400 MHz): δ 9.31 (s,1H),8.58 (s,1H),8.23 (d, J = 1.6 Hz,1H),8.21 (s,1H),7.96-7.90 (m,2H),6.87 (q, J = 5.6 Hz,1H),4.96-4.89 (m,1H),4.17 (q, J = 7.2 Hz,2H),1.58 (d, J = 5.2 Hz,3H),1.51 (d, J = 6.4 Hz,6H),1.23 (t, J = 6.8 Hz,3H). MS (ESI, m/z):411.1 [M+H]⁺.

### Example 8: Synthesis of (2-acetoxy)ethyl 1-(3-cyano-1-isopropyl-1H-indol-5-yl)-1H-pyrazole-4-carboxylate (12)

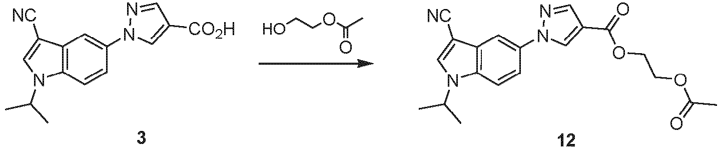

Compound **3** and ethylene glycol monoacetate were used as raw materials, and reference can be made to Example 4 for the experimental procedure for synthesizing compound **12.** ¹H NMR (DMSO-d₆,400 MHz): δ 9.25 (s,1H),8.58 (s,1H),8.22 (d, J = 1.6 Hz,1H),8.16 (s,1H),7.96-7.90 (m,2H),4.96-4.89 (m,1H),4.46-4.44 (m,2H),4.34-4.32 (m,2H),2.05 (s,3H),1.51 (d, J = 6.8 Hz,6H). MS (ESI, m/z):381.1 [M+H]⁺.

### Example 9: Synthesis of (2-methoxy)ethyl 1-(3-cyano-1-isopropyl-1H-indol-5-yl)-1H-pyrazole-4-carboxylate (13)

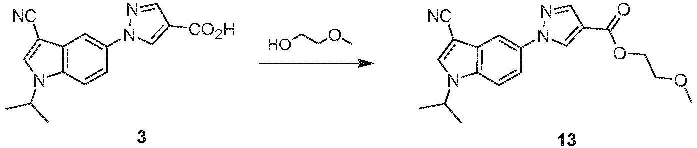

Compound **3** and ethylene glycol monomethyl ether were used as raw materials, and reference can be made to Example 4 for the experimental procedure for synthesizing compound **13.** ¹H NMR (DMSO-d₆,400 MHz): δ 9.23 (s,1H),8.58 (s,1H),8.22 (d, J = 2.0 Hz,1H),8.15 (s,1H),7.96-7.89 (m,2H),4.96-4.89 (m,1H),4.38 (t, J = 4.8 Hz,2H),3.65 (t, J = 4.8 Hz,2H),3.32 (s,3H),1.51 (d, J = 6.8 Hz,6H). MS (ESI, m/z):353.1 [M+H]⁺.

### Example 10: Synthesis of cinnamyl1-(3-cyano-1-isopropyl-1H-indol-5-yl)-1H-pyrazole-4-carboxylate (14)

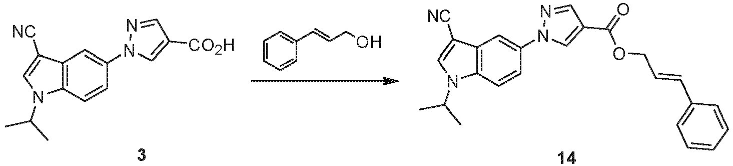

Compound **3** and cinnamyl alcohol were used as raw materials, and reference can be made to Example 4 for the experimental procedure for synthesizing compound **14.** ¹H NMR (DMSO-d₆,400 MHz): δ 9.29 (s,1H),8.57 (s,1H),8.23-8.21 (m,2H),7.97-7.90 (m,2H),7.52-7.50 (m,2H),7.38-7.29 (m,3H),6.81 (d, J = 16.0 Hz,1H),6.53-6.48 (m,1H),4.95-4.91 (m,3H),1.49 (d, J = 6.4 Hz,6H). MS (ESI, m/z):411.1 [M+H]⁺.

### Example 11: Synthesis of (1-isobutyryloxy)ethyl 1-(3-cyano-1-isopropyl-1H-indol-5-yl)-1H-pyrazole-4-carboxylate (15)

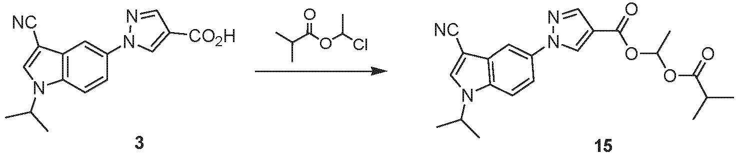

Compound **3** and 1-chloroethyl isobutyrate were used as raw materials, and reference can be made to Example 7for the experimental procedure for synthesizing compound 15. ¹H NMR (DMSO-d₆,400 MHz): δ 9.28 (s, 1H),8.57 (s,1H),8.22 (d, J = 1.6 Hz,1H),8.19 (s,1H),7.96-7.90 (m,2H),6.97 (q, J = 5.6 Hz,1H),4.96-4.89 (m,1H),2.61-2.54 (m,1H),1.55 (d, J = 5.6 Hz,3H),1.51 (d, J = 6.8 Hz,6H),1.12-1.09 (m,6H). MS (ESI, m/z):409.2 [M+H]⁺.

### Example 12: Synthesis of (5-methyl-2-oxo-1,3-dioxol-4-yl)methyl 2-(3-cyano-1-isopropyl-1H-indol-5-yl)-2H-1,2,3-triazole-4-carboxylate (17)

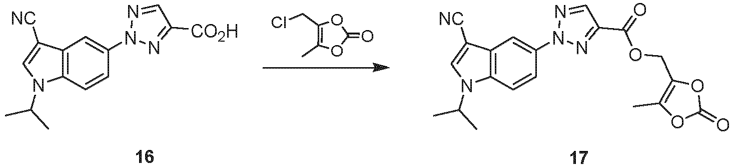

2-(3-cyano-1-isopropyl-1H-indol-5-yl)-2H-1,2,3-triazole-4-carboxylic acid **(16)** (reference can made to Examples 2 and 3 in patent CN 115160299 for experimental procedure for synthesizing compound **16)** and 4-chloromethyl-5-methyl-1,3-dioxol-2-one were used as raw materials, and reference can be made to step D in Example 1 for the experimental procedure for synthesizing compound **17.** ¹H NMR (CDCl₃,400 MHz): δ 8.56 (d, J = 2.0 Hz, 1H), 8.29 (s, 1H), 8.17 (dd, J = 2.0, 8.8 Hz, 1H), 7.82 (s, 1H), 7.56 (d, J = 8.8 Hz, 1H), 5.18 (s, 2H), 4.79-4.73 (m, 1H), 2.29 (s, 3H), 1.62 (d, J = 6.4 Hz, 6H). MS (ESI, m/z):408.0 [M+H]⁺.

### Example 13: Synthesis of bis[2-(3-cyano-1-isopropyl-1H-indol-5-yl)-2H-1,2,3-triazole-4-carboxylic acid]propan-1,3-diester (18)

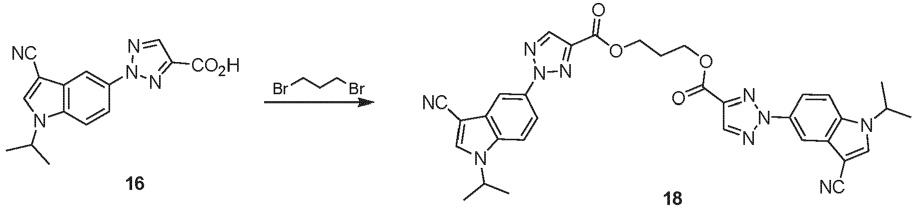

Compound **16** and 1,3-dibromopropane were used as raw materials, and reference can be made to Example 2 for the experimental procedure for synthesizing compound **18.** ¹H NMR (CDCl₃,400 MHz): δ 8.44 (d, J = 2.0 Hz,2H),8.25 (s,2H),8.10 (dd, J = 2.0,8.8 Hz,2H),7.79 (s,2H),7.53 (d, J = 8.8 Hz,2H),4.79-4.72 (m,2H),4.65 (t, J = 6.0 Hz,4H),2.39 (t, J = 6.0 Hz,2H),1.61 (d, J = 6.8 Hz,12H). MS (ESI, m/z):631.2 [M+H]⁺.

### Example 14: Synthesis of {1-[(ethoxycarbonyl)oxy]}ethyl 2-(3-cyano-1-isopropyl-1H-indol-5-yl)-2H-1,2,3-triazole-4-carboxylate (19)

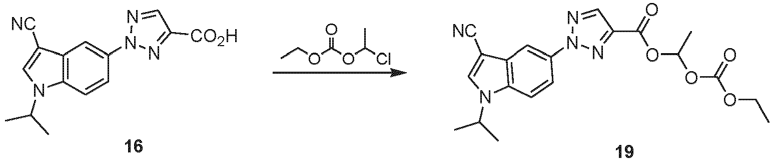

Compound **16** and 1-chloroethyl ethyl carbonate were used as raw materials, and reference can be made to Example 7 for the experimental procedure for synthesizing compound **19.** ¹H NMR (CDCl₃,400 MHz): δ 8.56 (d, J = 2.0 Hz, 1H), 8.28 (s, 1H), 8.17 (dd, J = 2.0, 8.8 Hz, 1H), 7.81 (s, 1H), 7.55 (d, J = 8.8 Hz, 1H), 7.10 (q, J = 5.2 Hz, 1H), 4.79-4.72 (m, 1H), 4.26 (q, J = 7.2 Hz, 2H), 1.72 (d, J = 5.6 Hz, 3H), 1.61 (d, J = 6.8 Hz, 6H), 1.34 (t, J = 6.8 Hz, 3H). MS (ESI, m/z):412.1 [M+H]⁺.

### Example 15: Synthesis of (3,7,11-trimethyldodecyl-2,6,10-trien-1-yl)2-(3-cyano-1-isopropyl-1H-indol-5-yl)-2H-1,2,3-triazole-4-carboxylate (20)

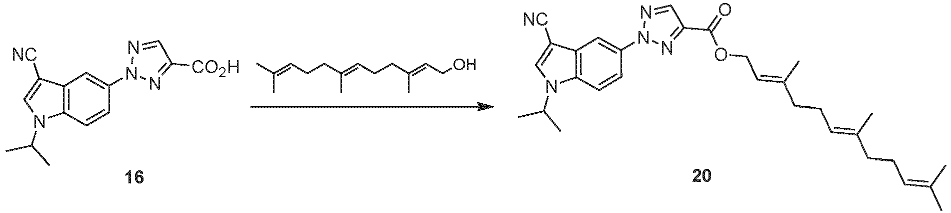

Compound **16** and 3,7,11-trimethyldodecyl-2,6,10-trien-1-ol were used as raw materials, and reference can be made to Example 4 for the experimental procedure for synthesizing compound **20.** ¹H NMR (DMSO-d₆,400 MHz): δ 8.65 (s,1H),8.61-8.60 (m,1H),8.24 (s,1H),8.06 (dd, J = 2.0,8.8 Hz,1H),8.00 (d, J = 8.8 Hz,1H),5.50-5.43 (m,1H),5.09-4.84 (m,5H),2.10-1.87 (m,8H),1.76 (s,3H),1.63-1.61 (m,2H),1.56-1.49 (m,13H). MS (ESI, m/z):544.3 [M+HCO₂]⁻.

### Example 16: Synthesis of (pyridin-2-yl)methyl 2-(3-cyano-1-isopropyl-1H-indol-5-yl)-2H-1,2,3-triazole-4-carboxylate (21)

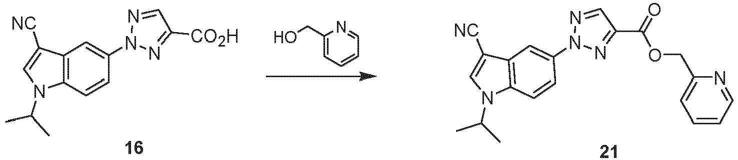

A mixture comprising compound **16** (80 mg, 0.271 mmol), pyridine-2-methanol (36 mg, 0.330 mmol), DCC (84 mg, 0.407 mmol), DMAP (3 mg, 0.0246 mmol) and dichloromethane (5 mL) was stirred at room temperature overnight. Insolubles were removed by filtration, and the filter cake was rinsed with dichloromethane (5 mL). The solvent was evaporated under reduced pressure. The product was purified by column chromatography (200-300 mesh silica gel, dichloromethane elution) to give(pyridin-2-yl)methyl 2-(3-cyano-1-isopropyl-1H-indol-5-yl)-2H-1,2,3-triazole-4-carboxylate **(21)**(69 mg). The yield was 65.9%. ¹H NMR (CDCl₃,400 MHz): δ 8.65-8.64 (m,1H),8.57 (d, J = 2.0 Hz,1H),8.32 (s,1H),8.18 (dd, J = 2.0,8.8 Hz,1H),7.81 (s,1H),7.78-7.74 (m,1H),7.57-7.50 (m,2H),7.30-7.28 (m,1H),5.58 (s,2H),4.79-4.72 (m,1H),1.61 (d, J = 6.8 Hz,6H). MS (ESI, m/z):387.4 [M+H]⁺.

### Example 17: Synthesis of methyl (5-methyl-2-oxo-1,3-dioxol-4-yl)2-(3-cyano-1-isopropyl-1H-indol-5-yl)isonicotina te(27)

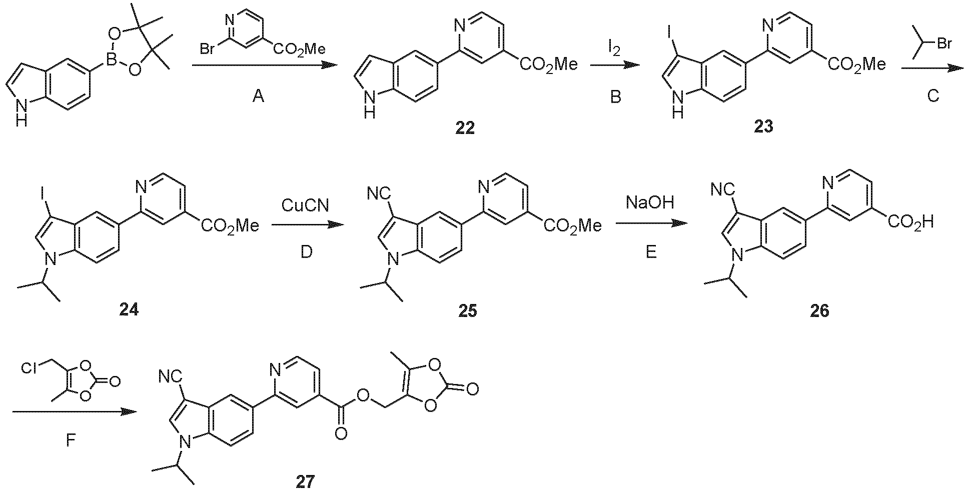

Step A: To a mixture comprising 5-indoleboronic acid pinacol ester (7.29 g, 30.0 mmol), methyl 2-bromopyridine-4-carboxylate (7.78 g, 36.0 mmol), potassium carbonate (10.4 g, 75.2 mmol), dioxane (100 mL) and water (20 mL), [1,1'-bis(diphenylphosphino)ferrocene]palladium dichloride (1.09 g, 1.50 mmol) was added. After the addition was complete, the resulting mixture was stirred at 80°C under nitrogen for 3 hours. Most of the solvent was evaporated under reduced pressure. Water (100 mL) was added. Ethyl acetate (100 mL x 3) was used for extraction. The combined organic phase was washed with saturated brine (50 mL), and dried over anhydrous sodium sulfate. The solvent was evaporated under reduced pressure. The product was purified by column chromatography (200-300 mesh silica gel, ethyl acetate: petroleum ether=1:5 elution) to give methyl 2-(1H-indol-5-yl)isonicotinate **(22)** (1.30 g). The yield was 17.2%.

Step B: To a solution of compound **22** (1.30 g, 5.15 mmol) in DMF (25 mL), cesium carbonate (3.35 g, 10.3 mmol) and iodine (2.62 g, 10.3 mmol) were added. After the addition was complete, the resulting mixture was stirred overnight at room temperature. Water (60 mL) and a 2M sodium thiosulfate solution (20 mL) were added. The filter cake was dissolved with ethyl acetate (200 mL), insolubles were removed by means of filtration, and then the resultant was dried over anhydrous sodium sulfate. The solvent was evaporated under reduced pressure to give methyl 2-(3-iodo-1H-indol-5-yl)isonicotinate **(23)** (1.10 g). The yield was 51.4%. MS (ESI, m/z):379.1 [M+H]⁺.

Step C: A mixture comprising compound **23** (1.10 g, 2.91 mmol), potassium carbonate (480 mg, 3.47 mmol), bromoisopropane (530 mg, 4.31 mmol), potassium iodide (100 mg, 0.602 mmol) and DMF (20 mL) was stirred overnight at 60°C, and cooled to room temperature. Water (80 mL) was added. Ethyl acetate (40 mL x 3) was used for extraction. The combined organic phase was washed successively with water (30 mL x 2) and saturated brine (30 mL), and dried over anhydrous sodium sulfate. The solvent was evaporated under reduced pressure. The product was purified by column chromatography (200-300 mesh silica gel, ethyl acetate: petroleum ether=1:10) to give methyl 2-(3-iodo-1-isopropyl-1H-indol-5-yl)isonicotinate (24)(600 mg). The yield was 49.1%.

Step D: A mixture comprising compound **24** (600 mg, 1.43 mmol), cuprous cyanide (200 mg, 2.33 mmol) and DMF (6 mL) was stirred at 120°C overnight, and cooled to room temperature. Ethyl acetate (30 mL) and water (20 mL) were added, and insolubles were removed by means of filtration. The combined organic layer was washed with water (15 mL x 2) and saturated brine (15 mL), and dried over anhydrous sodium sulfate. The solvent was evaporated under reduced pressure to give methyl 2-(3-cyano-1-isopropyl-1H-indol-5-yl)isonicotinate (25%) (267 mg). The yield was 58.5%.

Step E: A mixture comprising compound **25** (267 mg, 0.836 mmol), a 2M sodium hydroxide solution (4 mL), methanol (1.3 mL) and THF (1.3 mL) was stirred at room temperature for 30 minutes. Water (15 mL) was added, ethyl acetate (10 mL) was used for extraction, and the product was in the aqueous phase. The pH of the aqueous phase was adjusted to 5-6 bya 2M citric acid solution. Filtration was performed to give 2-(3-cyano-1-isopropyl-1H-indol-5-yl)isonicotinate **(26)** (255 mg). The yield was 99.9%.

Reference can be made to step D in Example 1 for the experimental procedure of Step F, and methyl (5-methyl-2-oxo-1,3-dioxol-4-yl)2-(3-cyano-1-isopropyl-1H-indol-5-yl)isonicotinate (27) was obtained. ¹H NMR (CDCl₃,400 MHz): δ 8.88 (d, J = 5.2 Hz, 1H), 8.44 (d, J = 1.6 Hz, 1H), 8.36 (s, 1H), 8.11 (dd, J = 1.6, 8.8 Hz, 1H), 7.78-7.77 (m, 2H), 7.57 (d, J = 8.8 Hz, 1H), 5.19 (s, 2H), 4.80-4.73 (m, 1H), 2.30 (s, 3H), 1.61 (d, J = 6.4 Hz, 6H). MS (ESI, m/z):418.2 [M+H]⁺.

### Example 18: Synthesis of ethyl {1-[(ethoxycarbonyl) oxy]}2-(3-cyano-1-isopropyl-1H-indol-5-yl)isonicotinate (28)

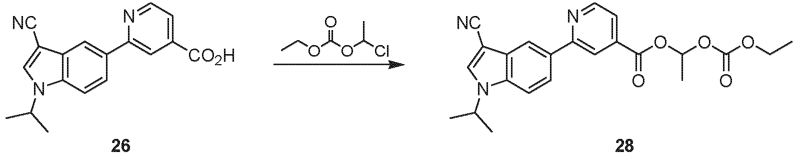

Compound **26** and 1-chloroethyl ethyl carbonate were used as raw materials, and reference can be made to Example 7 for the experimental procedure for synthesizing compound **28.** ¹H NMR (CDCl₃,400 MHz): δ 8.86 (d, J = 4.8 Hz, 1H), 8.44 (d, J = 1.6 Hz, 1H), 8.36 (s, 1H), 8.09 (dd, J = 1.6, 8.8 Hz, 1H), 7.80-7.78 (m, 2H), 7.56 (d, J = 8.8 Hz, 1H), 7.09 (q, J = 5.2 Hz, 1H), 4.80-4.73 (m, 1H), 4.26 (q, J = 7.2 Hz, 2H), 1.72 (d, J = 5.6 Hz, 3H), 1.61 (d, J = 6.8 Hz, 6H), 1.34 (t, J = 7.2 Hz, 3H). MS (ESI, m/z):422.4 [M+H]⁺.

### Example 19: synthesis of [4-(nitrooxy)]butyl 1-(3-cyano-1-isopropyl-1H-indol-5-yl)-1H-pyrazole-4-carboxylate (30)

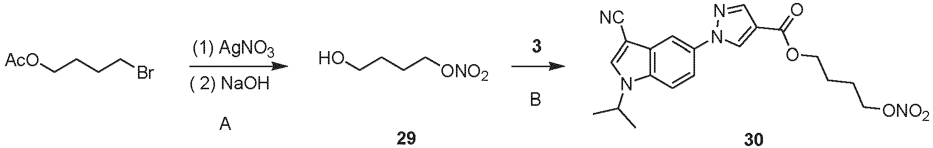

Step A: A mixture comprising 4-bromobutyl acetate (1.0 g, 5.13 mmol), silver nitrate (1.30 g, 7.65 mmol) and acetonitrile (15 mL) was stirred under reflux overnight in the dark, cooled to room temperature, and filtered to remove insolubles. Water (60 mL) was added. Ethyl acetate (30 mL x 3) was used for extraction. The combined organic phase was washed with saturated brine (20 mL) and dried over anhydrous sodium sulfate. The solvent was evaporated under reduced pressure, and then a 2 M sodium hydroxide solution (2.5 mL) and methanol (5 mL) were added to the residue. After the addition was complete, the resulting mixture was stirred at room temperature for 2 hours. Water (20 mL) was added. Ethyl acetate (20 mL x 2) was used for extraction. The combined organic phase was washed with saturated brine (10 mL) and dried over anhydrous sodium sulfate. The solvent was evaporated under reduced pressure. The product was purified by column chromatography (200-300 mesh silica gel,petroleum ether: ethyl acetate=5:1 elution) to give (4-hydroxy)butyl nitrate (29)(400 mg). The yield was 57.5%.

Reference can be made to Example 4 for the experimental procedure of Step B, and [4-(nitrooxy)]butyl 1-(3-cyano-1-isopropyl-1H-indol-5-yl)-1H-pyrazole-4-carboxylate (30) was obtained. ¹H NMR (DMSO-d₆,400 MHz): δ 9.22 (s,1H),8.57 (s,1H),8.20 (d, J = 1.6 Hz,1H),8.16 (s,1H),7.95-7.89 (m,2H),4.96-4.89 (m,1H),4.61 (t, J = 6.0 Hz,2H),4.28 (t, J = 6.0 Hz,2H),1.87-1.78 (m,4H),1.51 (d, J = 6.8 Hz,6H). MS (ESI, m/z):412.5 [M+H]⁺.

### Example 20: Synthesis of phenyl [3-(nitrooxy)methyl]1-(3-cyano-1-isopropyl-1H-indol-5-yl)-1H-pyrazole-4-carbox ylate (32)

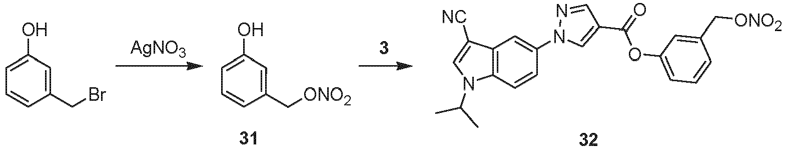

Step A: A mixture comprising m-hydroxybenzyl bromide (500 mg, 2.67 mmol), silver nitrate (500 mg, 2.94 mmol) and acetonitrile (5 mL) was stirred in ice-water bath for 5 hours in the dark. Insolubles were removed by filtration. Water (20 mL) was added. Ethyl acetate (20 mL x 2) was used for extraction. The combined organic phase was washed with saturated brine (10 mL) and dried over anhydrous sodium sulfate. The solvent was evaporated under reduced pressure. The product was purified by column chromatography (200-300 mesh silica gel,petroleum ether: ethyl acetate=35:1 elution) to give (3-hydroxy)benzyl nitrate **(31)** (230 mg). The yield was 50.9%.

Reference can be made to Example 4 for the experimental procedure of Step B, and phenyl [3-(nitrooxy)methyl] 1-(3-cyano-1-isopropyl-1H-indol-5-yl)-1H-pyrazole-4-carboxylate **(32)** was obtained. ¹H NMR (DMSO-d₆,400 MHz): δ 9.49 (s,1H),8.58 (s,1H),8.36 (s,1H),8.27 (s,1H),7.98-7.94 (m,2H),7.57-7.53 (m,1H),7.44-7.35 (m,3H),5.64 (s,2H),4.97-4.90 (m,1H),1.52 (d, J = 6.4 Hz,6H). MS (ESI, m/z):446.1 [M+H]⁺.

### Example 21: Experimental study of the compounds for treating hyperuricemia in rats

### 1. Experimental materials

### (1) Test drugs

Compound**30** and **32**were light yellow powders and triturated with 0.5% CMC-Na just prior to use to preparesuspensionshaving a corresponding concentration for gavage.

Febuxostat, purchased from Sigma, was triturated with 0.5% CMC-Na just prior to use to prepare a suspension having a corresponding concentration for gavage.

### (2) Animal and Feeding

### a. Animal species and source

Sprague Dawley (SD) rats, SPF grade, 30, male, weighing 230-250 g, purchased from Shanghai SLACLaboratory Animal Co., Ltd., permission number: SCXK (hu) 2022-0004, quality certificationnumber: 20220004017238.

### b. Feeding conditions

The rats were all housed in independent ventilation cages with an air cleanliness level of 10000. The laboratory temperature: 26 ± 2°C; the relative humidity: 60%-80%; the air exchange frequency per hour: 10-15 times/hour; the light cycle: 12 (day)/12 (night) hours, 3 rats per cage.

Feed: complete pelleted feed for rats, purchased from Jiangsu Xietong Pharmaceutical Bio-engineering Co., Ltd., and its quality complying with GB14924.1-2001 "General Quality Standard for Compound Feed for Laboratory Animals".

Bedding: sterilized granule pads purchased from Jiangsu Xietong Pharmaceutical Bio-engineering Co., Ltd.

Water: purified drinking water, free drinking after acidification.

### (3) Main instruments and equipment

Varioskan LUX multifunctional microplate reader purchased from Thermo, USA; BS210S precision electronic balance (0.1 mg-10 g) purchased from Sartorius, Germany; FEJ-200 electronic balance (0.1-200 g) purchased from Fuzhou FuriWeightingElectronics Co., Ltd.; Pacific TII+Genpure XCAD PLUS UV/TOC/UF pure water ultrapure water system purchased from Thermo, USA.

### (4) Main reagents

Uric acid assaykit (phosphotungstic acid reduction method), batch number: 20230224, purchased from NanJing Jiancheng Bioengineering Institute; Potassium oxonate, article number: 00164, batch number: T6GKM-TA, purchased from Tokyo Chemical Industry Co. , Ltd., Japan (TCI); carboxymethyl cellulose sodium (CMC-Na), batch number: 20170810, chemically pure, purchased from Sinopharm chemical reagent Co., Ltd.

### 2. Experimental methods

### (1) Grouping

30 male SD rats weighed approximately 220-240 g after one week of adaptation. They were randomly divided into 5groups according to the body weight, with 6rats in each group, namely: (1) normal group (0.5% CMC-Na), (2) model group (0.5% CMC-Na), (3) Febuxostat, 2 mg/kg, (4) compound **30,** 2 mg/kg and(5) compound **32,** 2 mg/kg. The drugs in each group were prepared into a corresponding concentrationsuspension, and the administration volume was 0.5 mL/100 g.

### (2) Model establishment, dosing regimen, and detection indicators

After the rats in each group were purchased and adapted to feeding, they were fasted for 12 h, and intraperitoneally injected with potassium oxonate at a dose of 300 mg/kg to establish a model. 0.5 h after modeling, each test drug group was subjected to gavage administration once. The drug was administered continuously for 3 days. On day 3, blood samples werecollected from the retro-orbital venous plexus before and 1, 3 and 5 h after injection of potassium oxonate. The samples were centrifuged at 3500 rpm for 10 min, and 30 µL of serum was collected to measure the uric acid level at each time point.

### (3) Data processing and statistical methods

The measurement data of each test were expressed as (mean) ±(standard deviation). The comparison between groups used ANOVA-Dunnett T test to examine the significance. P < 0.05 was used as the significance index, and P < 0.01 was used as the extremely significant index.

### 3. Experimental results

Compared with the normal group, the serum uric acid level in the potassium oxonate model group increased significantly at 1, 3 and 5 h after modeling (P<0.05). Compared with the model group at the same time point, Febuxostat can significantly reduce the serum uric acid level after modeling (P<0.01). Compared with the model group at the same time point, compound **30**and compound **32** can significantly reduce the serum uric acid level after modeling(P<0.01 or P<0.05). The results are shown in Table 1.

**Table 1. Effect on serum uric acid level in potassium oxonate-induced hyperuricemic rats after 3-day administration (x̅ ± s)**

| Group | Dose (mg/kg) | Number of animals | Uric acid (µmol/L) | | | |
|---|---|---|---|---|---|---|
| | | | 0 h | 1h | 3 h | 5 h |
| normal group | - | 6 | 46.8 ± 6.0 | 52.0 ± 9.1 | 55.7 ± 6.4 | 56.0 ± 9.4 |
| model group | - | 6 | 49.3 ± 12.3 | 86.6 ± 11.1 ^{##} | 120.8 ± 20.2^{##} | 107.2 ± 27.2^{##} |
| Febuxostat | 2 | 6 | 39.2 ± 6.2 | 34.4 ± 7.1** | 31.2 ± 7.6** | 24.7 ± 4.7** |
| compound **30** | 2 | 6 | 45.0 ± 8.4 | 70.0 ± 14.8* | 94.2 ± 22.7* | 72.2 ± 11.4* |
| compound **32** | 2 | 6 | 49.1 ± 6.7 | 68.9 ± 15.8* | 82.2 ± 23.3** | 65.8 ± 13.6** |

| | | | | | | |
|---|---|---|---|---|---|---|
| ^{##}P< 0.01, compared with the solvent group at the same time point; *P<0.05, **P<0.01, compared with the model group at the same time point. | | | | | | |

### Example 22: In vivo pharmacokinetic experiments of compound 11 in SD rats

### 1. Experimental materials

### (1) Test drugs

Preparation of compound stock solution: An appropriate amount of compound solid powder was be weighed and added in a certain amount of DMSO, and vortex ultrasound to obtain a 10 mg/mL stock solution.

Preparation of test compound for gavage: respectively taking an appropriate amount of the compound stock solution, adding a certain amount of Solutol HS15 solution, vortexing for 1 minute, then adding a certain amount of physiological saline, and fully mixing to obtain a 1 mg/mL solution.

Preparation of test compound for intravenous injection: respectively taking an appropriate amount of the compound stock solution, adding a certain amount of Solutol HS15 solution, vortexing for 1 minute, then adding a certain amount of physiological saline, and fully mixing until uniform, so as to obtain a 0.5 mg/mL solution.

### (2) Experimental animal

SD rats, male, SPF grade, 6-8 weeks old, purchased from JH Laboratory Animal Co. LTD. Permission number: SCXK (SH) 2022-0009, certification number: 20220009004139.

### 2. Experimental methods

### (1) Dose and administration

The experimental animals were fasted overnight before gavage administration, and were given food 4 hours after administration, with free access to water. Each test compound was divided into two groups, namely, intravenous administration group and oral administration group. The specific dosage and method of administration are shown in Table 2 below.

**Table 2 Dose and administration of compound 11 to SD rats**

| **Group** | **Numbe r of animals** | **Dose of administratio n (mg/kg)** | **Volume of administratio n (mL/kg)** | **Concentratio n (mg/mL)** | **Route of administratio n** |
|---|---|---|---|---|---|
| Intravenous administratio n group | 3 | 1 | 2 | 0.5 | intravenous injection |
| Oral administratio n group | 3 | 10 | 10 | 1 | oral |

### (2) Experimental procedure

Blood samples (150 µL/sample) were collected from the jugular vein of SD rats before administration and at 5 min (only intravenous administration group), 15 min, 30 min, 1 h, 2 h, 4 h, 8 h and 24 h after administration, respectively. The samples were placed in centrifuge tubes containing anticoagulant sodium heparin and centrifuged at 4°C, 2000 g for 5 minutes to separate plasma. The plasma samples were analyzed by LC/MS/MS to detect the concentration of each test compound.

### (3) Pharmacokinetic analysis

Non-compartmental model-related parameters were calculated by WinNonlin^{®} Professional software.

### 3. Experimental results

The pharmacokinetic parameters of the tested compounds in SD rats obtained according to the method above are shown in Table 3. The pharmacokinetic parameters of the compounds of the present invention are good and the bioavailability is high.

**Table 3. Pharmacokinetic parameters of each compound in SD rats after oral or intravenous administration**

| **Gavage administration - 10 mg/kg** | | | | | | |
|---|---|---|---|---|---|---|
| **Compound number** | **t_{1/2} (h)** | **Tₘₐₓ (h)** | **Cₘₐₓ (ng/mL)** | **AUC_{INF} (hr*ng/mL )** | **MRT_{INF} (h)** | |
| **11** | 3.44 | 0.250 | 4670 | 12363 | 4.22 | |

| **Intravenous injection administration - 1 mg/kg** | | | | | | |
|---|---|---|---|---|---|---|
| **Compound number** | **t_{1/2} (h)** | **Tₘₐₓ (h)** | **Cₘₐₓ (ng/mL)** | **AUC_{INF} (hr*ng/mL)** | | **MRT_{INF} (h)** |
| **11** | 7.72 | 0.083 | 1860 | 1099 | | 3.69 |

## Claims

1. A compound as represented by general formula (I) or a pharmaceutically acceptable salt thereof, 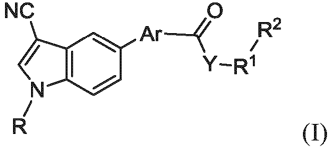 wherein,
R is C₁₋₆ alkyl, substituted C₁₋₆ alkyl, C₃₋₆ cycloalkyl, substituted C₃₋₆ cycloalkyl, C₃₋₆ heterocycloalkyl or substituted C₃₋₆ heterocycloalkyl; wherein the substituent in each group involved in R is one or more selected from deuterium, cyano, nitro, halo, C₁₋₆ alkyl, C₁₋₆ alkoxy, C₃₋₆ cycloalkyl or C₃₋₆ heterocycloalkyl;
Ar is a substituted or unsubstituted group of: 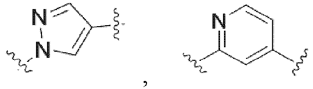
or 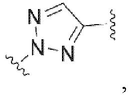 and the substituent in the Ar group is one or more selected from deuterium, hydroxyl, halo, C₁₋₄alkyl or C₁₋₄alkoxy;
Y is O or NR³,
R¹ is a linking bond or substituted or unsubstituted C₁₋₆ alkylene or substituted or unsubstituted C₂₋₁₂ alkenylene, and the substituent in the R¹ group is one or more selected from deuterium, hydroxyl, amino, cyano, halo, C₁₋₄ alkyl or C₁₋₄ alkoxy;
R² is one or more of hydrogen, nitrooxy, carboxyl or the following substituted or unsubstituted groups: dioxol-2-one, C₄₋₁₂fused heteroarylcyclyl, C₄₋₁₆ fused heteroarylcyclylpyrazolylcarbonyloxy, C₄₋₁₆ fused heteroarylcyclylpyridylcarbonyloxy, C₄₋₁₆ fused heteroarylcyclyltriazolylcarbonyloxy, C₂₋₆ ester, pyridyl, phenyl, C₁₋₆ alkoxy, C₂₋₂₀ alkenyl, C₂₋₂₀ alkynyl, C₂₋₈ alkylcarbonyloxy or C₂₋₈ alkoxycarbonyloxy, and the substituent in the R² group is one or more selected from deuterium, hydroxyl, amino, cyano, halo, C₁₋₄ alkyl, halo C₁₋₄ alkyl, nitrooxy-substituted C₁₋₄ alkyl or C₁₋₄ alkoxy; and the C₄₋₁₆ fused heteroarylcyclyl in the R² group does not contain indazolyl; and
R³ is hydrogen or C₁₋₆ alkyl.

2. The compound or the pharmaceutically acceptable salt thereof according to claim 1, wherein the compound is selected from compounds as represented by general formula (II), (III) or (IV):

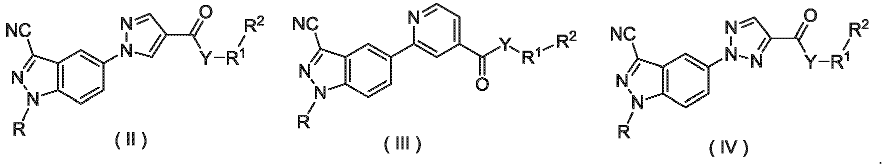

3. The compound or the pharmaceutically acceptable salt thereof according to claim 1, wherein Y is O or NH, R is C₃₋₆ alkyl, substituted C₁₋₆ alkyl, C₃₋₆ cycloalkyl, substituted C₃₋₆ cycloalkyl, C₃₋₆ heterocycloalkyl or substituted C₃₋₆ heterocycloalkyl; wherein the substituent in each group involved in R is one or more selected from deuterium, cyano, nitro, halo, C₁₋₅ alkyl, C₁₋₅ alkoxy or C₃₋₆ cycloalkyl.

4. The compound or the pharmaceutically acceptable salt thereof according to claim 3, wherein R is C₃₋₆ alkyl, substituted C₁₋₆ alkyl, C₃₋₆ cycloalkyl, substituted C₃₋₆ cycloalkyl, tetrahydrofuran, substituted tetrahydrofuran, tetrahydrothiophene, substituted tetrahydrothiophene, tetrahydropyrrole or substituted tetrahydropyrrole; wherein the substituent in each group involved in R is one or more selected from deuterium, cyano, nitro, halo, C₁₋₅ alkyl, C₁₋₅ alkoxy or C₃₋₆ cycloalkyl.

5. The compound or the pharmaceutically acceptable salt thereof according to claim 1, wherein R¹ is a linking bond or substituted or unsubstituted C₁₋₄ alkylene or substituted or unsubstituted C₂₋₁₂ alkenylene, and the substituent in the R¹ group is one or more selected from deuterium, amino, cyano, halogen or C₁₋₄ alkoxy.

6. The compound or the pharmaceutically acceptable salt thereof according to claim 1, wherein R² is one or more of hydrogen, nitrooxy, carboxyl orthe following substituted or unsubstituted groups: dioxol-2-one, indazolyl, quinolinyl, isoquinolinyl, indolyl, benzofuranyl, purinyl, quinolinylpyrazolylcarbonyloxy, isoquinolinylpyrazolylcarbonyloxy, indolylpyrazolylcarbonyloxy, benzofuranylpyrazolylcarbonyloxy, purinylpyrazolylcarbonyloxy, quinolinylpyridylcarbonyloxy, isoquinolinylpyridylcarbonyloxy, indolylpyridylcarbonyloxy, benzofuranylpyridylcarbonyloxy, purinylpyridylcarbonyloxy, quinolyltriazolylcarbonyloxy, isoquinolyltriazolylcarbonyloxy, indolyltriazolylcarbonyloxy, benzofuranyltriazolylcarbonyloxy, purinyltriazolylcarbonyloxy, C₂₋₆ ester, pyridyl, phenyl, C₁₋₆ alkoxy, C₆₋₂₀ alkenyl, C₆₋₂₀ alkynyl, C₂₋₈ alkylcarbonyloxy or C₂₋₈ alkoxycarbonyloxy, and the substituent in the R² group is one or more selected from deuterium, hydroxyl, amino, cyano, halo, C₁₋₄ alkyl, halo C₁₋₄ alkyl, nitrooxy-substituted C₁₋₄ alkyl or C₁₋₄ alkoxy.

7. The compound or the pharmaceutically acceptable salt thereof according to claim 5, wherein R² is one or more of hydrogen, nitrooxy, carboxyl or the following substituted or unsubstituted groups: dioxol-2-one, indolylpyrazolylcarbonyloxy, indolylpyridylcarbonyloxy, indolyltriazolylcarbonyloxy, C₂₋₆ester, pyridyl, phenyl, C₁₋₆alkoxy, C₆₋₂₀alkenyl, C₂₋₈alkylcarbonyloxy or C₂₋₈alkoxycarbonyloxy, and the substituent in the R² group is one or more selected from deuterium, hydroxyl, amino, cyano, halo, C₁₋₄ alkyl, nitrooxy-substituted C₁₋₄ alkyl or C₁₋₄ alkoxy.

8. The compound or the pharmaceutically acceptable salt thereof according to claim 1, wherein the compound is selected from:

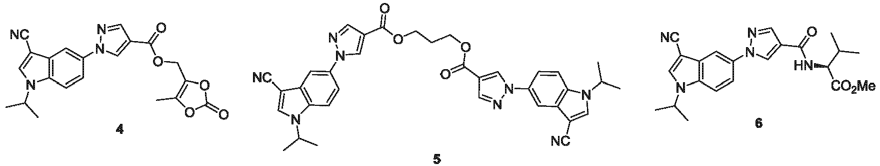

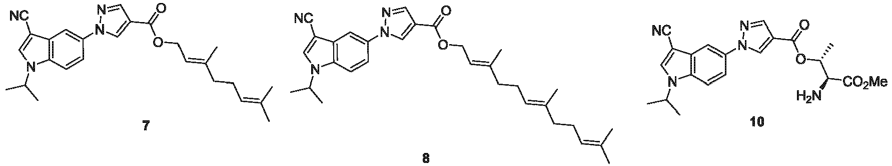

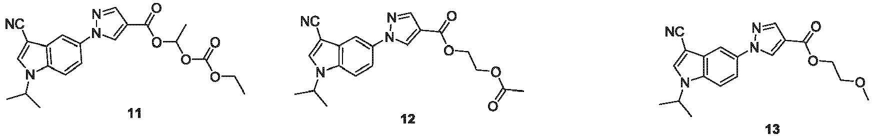

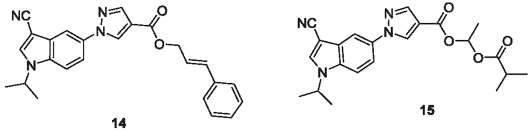

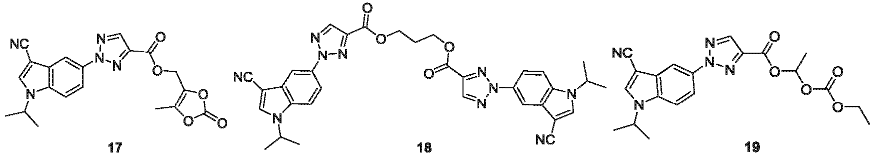

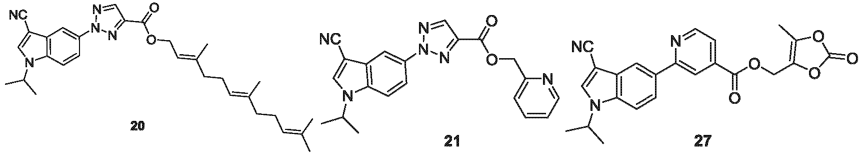

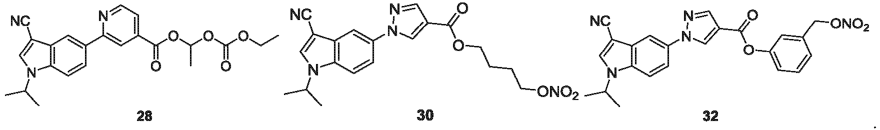

9. A pharmaceutical composition, comprising the compound or the pharmaceutically acceptable salt thereof of claim 1 as an active substance and pharmaceutically acceptable excipients.

10. Use of the compound or the pharmaceutically acceptable salt thereof of claim 1 in the preparation of an anti-gout medicament or an anti-hyperuricemia medicament.
